(19) Europäisches Patentamt
European Patent Office
Office européen des brevets

(11) Veröffentlichungsnummer: **0 196 270**
**B1**

(12) # EUROPÄISCHE PATENTSCHRIFT

(45) Veröffentlichungstag der Patentschrift: **10.05.89**

(51) Int. Cl.⁴: **C 07 J 1/00**, A 61 K 31/565

(21) Anmeldenummer: **86730051.9**

(22) Anmeldetag: **20.03.86**

(54) **Wässrige Kristallsuspension von Steroidglykoestern.**

(30) Priorität: **27.03.85 DE 3511588**

(43) Veröffentlichungstag der Anmeldung: **01.10.86 Patentblatt 86/40**

(45) Bekanntmachung des Hinweises auf die Patenterteilung: **10.05.89 Patentblatt 89/19**

(84) Benannte Vertragsstaaten: **AT BE CH DE FR GB IT LI LU NL SE**

(56) Entgegenhaltungen:
**EP-A-0 129 947**
**DE-A-2 558 076**

**STEROIDS, Band 41, Nr. 3, März 1983, Seiten 397-417, San Francisco, US; S. NADERI et al.: "Long-acting contraceptive agents: In vitro hydrolysis of esters of norethisterone and levonorgestrel"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Schulze, Paul- Eberhard, Dr., Endestrasse 30, D-1000 Berlin 39 (DE)**
Erfinder: **Acksteiner, Bernard, Dr., Ludwigkirchstrasse 9 a, D-1000 Berlin 15 (DE)**
Erfinder: **Düsterberg, Bernd, Dr., Spirdingseestrasse 27, D-1000 Berlin 49 (DE)**

EP 0 196 270 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft eine wäßrige Kristallsuspension eines 17-tertiären Steroidglykoesters, dadurch gekennzeichnet, daß sie eine Verbindung der allgemeinen Formel I

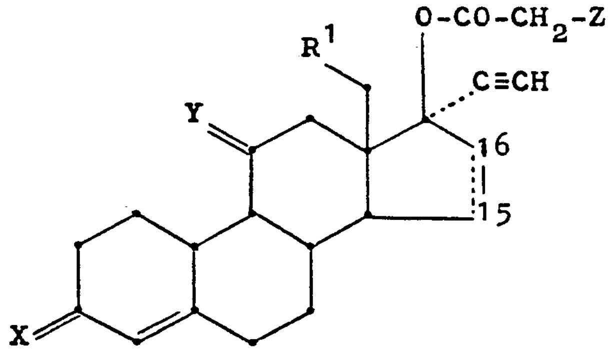

(I),

worin

X zwei Wasserstoffatome oder ein Sauerstoffatom,
Y zwei Wasserstoffatome oder eine Methylengruppe,
$R^1$ ein Wasserstoffatom oder eine Methylgruppe,
Z eine Hydroxygruppe oder die Gruppe -O-CO-$R^2$ mit $R^2$ in der Bedeutung einer Methyl- oder Phenylgruppe und
16┆15 eine C-C-Einfach- oder Doppelbindung

bedeuten,
in folgenden Fraktionen enthält:

0 - 30 Gewichtsprozent der Teilchengröße 3 - 15 μm,
40 - 90 Gewichtsprozent der Teilchengröße 15 - 60 μm und
20 - 60 Gewichtsprozent der Teilchengröße 30 - 100 μm.

Die Erfindung betrifft ferner Verbindungen der allgemeinen Formel I a

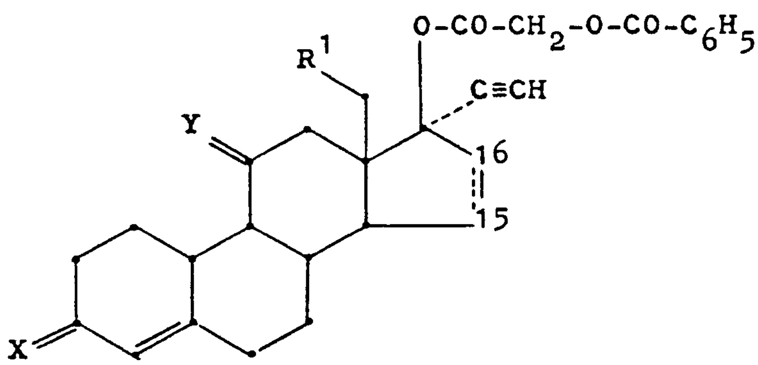

worin

X zwei Wasserstoffatome oder ein Sauerstoffatom,
Y zwei Wasserstoffatome oder eine Methylengruppe,
$R^1$ ein Wasserstoffatom oder eine Methylgruppe und
16┆15 eine C-C-Einfach- oder Doppelbindung

bedeuten, sowie Verfahren zu deren Herstellung, dadurch gekennzeichnet, daß man entsprechende Hydroxysteroide der allgemeinen Formel

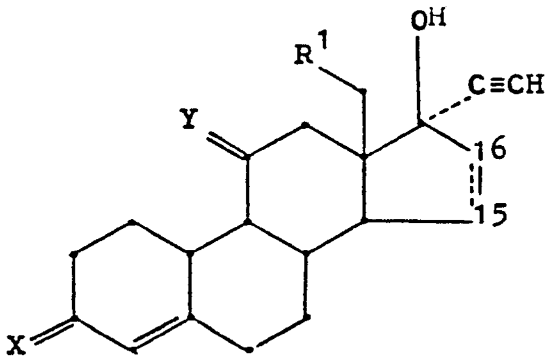

worin X, Y, $R^1$ und 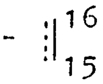 die oben angegebene Bedeutung haben, mit Benzoylglykoylchlorid verestert.

Die pharmakologische Wirkung der Steroide hält nur für kurze Zeit an. Als Kontrazeptiva müssen die Steroide täglich appliziert werden.

Es wurden bereits Versuche unternommen, die Wirkungsdauer durch Veresterung der Steroide zu verlängern. So ist bekannt, daß man einen protrahierenden Effekt erzielen kann, wenn man biologisch wirksame Steroidalkohole mit langkettigen, verzweigten oder cyclischen Fettsäuren verestert. Eine zusätzliche Depotwirkung kommt häufig dadurch zustande, daß man den Wirkstoff zu einer Trägersubstanz gibt, die den Wirkstoff verzögert abgibt. Ein injizierbares Depotkontrazeptivum ist zum Beispiel Norethisteronönanthat in öliger Lösung. Bei einer Dosierung von 200 mg Norethisteronönanthat in ml Rizinusöl/Benzylbenzoat im Verhältnis 6 : 4 hält die Wirkung etwa 12 Wochen an. Es zeigt sich jedoch, daß die Zahl der Schwangerschaften unter Norethisteronönanthat etwas größer ist als bei der täglichen oralen Tabletteneinnahme und daß unerwünschte Schwangerschaften insbesondere kurz vor Ende des Injektionsintervalls auftreten.

Wenn man die Serumsteroidkonzentration nach einmaliger intramuskulärer Injektion von 200 mg Norethisteronönanthat beim Menschen untersucht, findet man eine hohe Anfangskonzentration an Norethisteron, die zunächst sehr rasch und dann langsam weiter abfällt (Contraception 24 (1981) 15 - 17). Um einen bestimmten Norethisteronspiegel über 12 Wochen aufrechtzuerhalten, müssen physiologisch zu hohe Dosen an Norethisteronönanthat gegeben werden.

Auch nach Injektion von Medroxyprogesteronacetat als wäßrige Kristallsuspension werden hohe Anfangskonzentrationen von Medroxyprogesteron gemessen (J. Clin. Endocrinol. Metab. 44 (1977) 32 - 38).

In der deutschen Patentschrift 2 558 076 werden tertiäre 17-Acylglykolester beschrieben, die zum Beispiel durch Verestern des Steroidalkohols mit Glykolsäure und weiteres Verestern des primär erhaltenen Glykolesters mit einer langkettigen Carbonsäure entstehen. Eine weitere Herstellungsmöglichkeit besteht darin, daß der Steroidalkohol in einem Schritt mit dem gewünschten Acylglykoylchlorid umgesetzt wird. Durch Zwischenschaltung der Glykolsäure wird erreicht, daß der tertiäre 17-Ester in zwei Stufen fast vollständig verseift wird. Der stufenweise Aufbau des Esters führt aber nicht nur zu einer fast vollständigen Spaltung des Esters und Ausnutzung des Wirkstoffs, sondern auch zu einer raschen Bereitstellung des Wirkstoffs. Unmittelbar nach der Injektion wird für wenige Tage relativ viel Wirkstoff freigegeben, was zu Nebenwirkungen führen kann.

In der europäischen Patentanmeldung 129 947 werden Levonorgestrelester mit aliphatischen und cycloaliphatischen Säuren beschrieben. In wäßrigen Mikrokristallsuspensionen beträgt die Teilchengröße 3 bis 10 µm.

Es wurde nun gefunden, daß die 17-tertiären Steroidglykoester der allgemeinen Formel I in einer wäßrigen Mikrokristallsuspension zu einem gleichmäßigen zur Zyklusmitte leicht ansteigenden Steroidspiegel führen, der eine 4wöchige kontrazeptive Wirkung gewährleistet.

Eine Kristallsuspension von 50 mg Norethisteronglykobenzoat mit einer aus Abbildung 1 ersichtlichen prozentualen Verteilung der Kristallgrößen wurde am Pavian untersucht. Nach einmaliger intramuskulärer Injektion wurde ein über 4 Wochen ausreichend hoher, weitgehend gleichmäßig verlaufender Norethisteronspiegel im Plasma nachgewiesen (Abbildung 2). Aus dem Kurvenverlauf läßt sich schließen, daß der verabreichte Ester aus dem Depot vollständig freigegeben und in Norethisteron sowie die natürlichen Fettsäuren Glykolsäure und Benzoesäure gespalten wird. Die Freigabe des Esters und die Bildung von freiem Norethisteron erfolgen bis zu 5 Tagen mit fast konstanter Geschwindigkeit, um sich dann bis zum 11. Tag leicht zu erhöhen, bis zum 18. Tag auf das erste Niveau langsam abzusinken und nach 28 Tagen so niedrige Werte zu erreichen, daß eine Abbruchblutung eintritt. Es wird erkennbar, daß es sogar möglich erscheint, mit der geprüften Formulierung das "Drei-Stufen-Konzept" oraler Kontrazeptiva mit einem Injektionspräparat zu realisieren. Ein begrenzter Anstieg der Wirkstoffkonzentration in der Lutealphase ist zur "Bremsung" endogener Hormonsekretionen, die die Ovulation in der Zyklusmitte induzieren, als vorteilhaft zu bewerten.

Ähnliche Norethisteronspiegel werden mit Norethisteronglykolat und Norethisteronglykoacetat erhalten.

Als Steroide kommen Norethisteron und die vom Norethisteron abgeleiteten Steroide der allgemeinen Formel I infrage.

Bevorzugte Steroide der allgemeinen Formel I sind Norethisteron, Lynestrenol, Levonorgestrel, Gestoden und Desogestrel.

Während die entsprechenden Glykolate und Glykoacetate in der deutschen Patentschrift 2 558 076 beschrieben werden, sind die entsprechenden Glykobenzoate neu.

Zur Herstellung der neuen Steroidglykobenzoate der allgemeinen Formel I a werden zum Beispiel 4,56 mMol des Steroids in 7,5 ml Collidin und 7,5 ml Tetrachlorethylen unter Stickstoff gelöst und auf 120°C erhitzt. Danach tropft man innerhalb von 2 Stunden 4,9 mMol Benzoylglykoylchlorid in 10 ml Tetrachlorethylen hinzu. Die Base wird dann mit Oxalsäurelösung (aus 5 g Oxalsäure) extrahiert und die Lösung anschließend mit Natriumcarbonatlösung und Wasser ausgeschüttelt. Nach dem Trocknen über Natriumsulfat und Einengen an der Ölpumpe wird der Rückstand an Kieselgel mit Methylenchlorid/Aceton (bis 1 % Aceton) chromatographiert.

Man erhält zum Beispiel aus 1,36 g Norethisteron und 0,97 g Benzoylglykoylchlorid 450 mg Norethisteronglykobenzoat, das nach Umkristallisieren aus Diisopropylether bei 206 - 207°C schmilzt.

Die gute Kristallisationsfähigkeit der unter Formel I fallenden 17-tertiären Glykoester ist überraschend, da andere Glykoester einen unerwartet niedrigen Schmelzpunkt zeigen.

Nach bekannten Methoden wird eine Mikrokristallsuspension eines 17-tertiären Steroidglykoesters der allgemeinen Formel I hergestellt und so gesiebt, daß man 3 Fraktionen folgender Teilchengröße erhält:

a) 3 - 15 µm,
b) 15 - 60 µm und
c) 30 - 100 µm in etwa linearer Verteilung.

0 - 30 Gewichtsprozent a)
40 - 90 Gewichtsprozent b) und
20 - 60 Gewichtsprozent c)
werden etwa 5 Minuten im Wirbelstrom gemischt, mit physiologischer Kochsalzlösung, gegebenenfalls unter Zusatz eines Stabilisators wie Polyethylenstearat (Myrj 53®), aufgefüllt und hitzesterilisiert.

Die für eine Kontrazeption wirksame Dosis für einen Monat für den Menschen beträgt 20 - 50 mg des Steroids bzw. 30 - 75 mg des Glykoesters.

**Beispiel 1**

In einer aseptischen und pyrogenfreien Schallfix-Apparatur werden 5 g 17α-Ethinyl-17β-(O-benzoyl-glykoloyloxy)-4-estren-3-on in 50 ml Myrj®-Kochsalzlösung (0,9 gewichtsprozentige Kochsalzlösung mit 0,085 Gewichtsprozent Myrj®) 30 Minuten beschallt und die erhaltene Suspension fraktioniert gesiebt. Man entnimmt Fraktionen mit den Teilchengrößen

a) 5 - 15 µm,
b) 15 - 50 µm und
c) 35 - 50 µm.

Die Fraktionen a), b) und c) werden im Verhältnis 10 : 50 : 40 im Wirbelstrom gemischt und mit Myrj®-Kochsalzlösung (0,9 gewichtsprozentige Kochsalzlösung mit 0,085 Gewichtsprozent Myrj®) auf 100 ml aufgefüllt.

**Beispiel 2**

Unter gleichen Bedingungen wie in Beispiel werden 0,5 g Estradiol-17β-benzoyloxyacetat 3 Minuten beschallt. Man erhält eine Teilchenverteilung von

a) 15 Gewichtsprozent der Größe 5 - 10 µm,
b) 60 Gewichtsprozent der Größe 10 - 26 µm und
c) 25 Gewichtsprozent der Größe 26 - 40 µm.

Die Fraktionen a), b) und c) gemäß Beispiel 1 werden mit a), b) und c) gemäß Beispiel 2 in 100 ml Myrj®-Kochsalzlösung suspendiert und unter aseptischen und sterilen Bedingungen in Spritzbestecke mit je 1 ml Inhalt abgefüllt. Jedes Spritzbesteck enthält
50 mg 17α-Ethinyl-17β-(O-benzoyl-glykoloyloxy)-4-estren-3-on und
5 mg Estradiol-17β-benzoyloxyacetat zur intramuskulären Injektion als Kontrazeptivum für 28 Tage (1-Monatsspritze).

**Patentansprüche** für die Vertragsstaaten BE, CH, LI, DE, FR, GB, IT, LU, NL, SE

1. Wäßrige Kristallsuspension eines 17-tertiären Steroidglykoesters, dadurch gekennzeichnet, daß sie eine Verbindung der allgemeinen Formel I

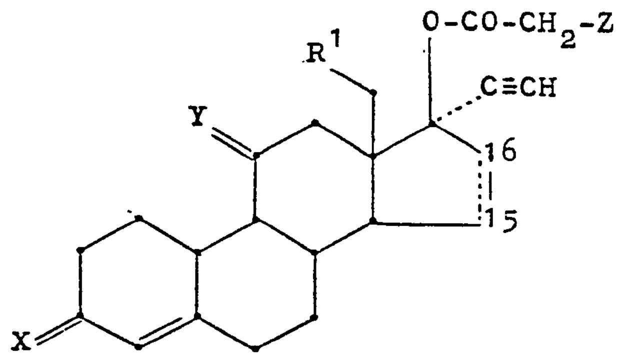

(I),

worin

X zwei Wasserstoffatome oder ein Sauerstoffatom,
Y zwei Wasserstoffatome oder eine Methylengruppe,
$R^1$ ein Wasserstoffatom oder eine Methylgruppe,
Z eine Hydroxygruppe oder die Gruppe -O-CO-$R^2$ mit $R^2$ in der Bedeutung einer Methyl- oder Phenylgruppe und
16 / 15 eine C-C-Einfach- oder Doppelbindung

bedeuten,
in folgenden Fraktionen enthält:

0 - 30 Gewichtsprozent der Teilchengröße 3 - 15 µm,
40 - 90 Gewichtsprozent der Teilchengröße 15 - 60 µm und
20 - 60 Gewichtsprozent der Teilchengröße 30 - 100 µm.

2. Verbindungen der allgemeinen Formel I a

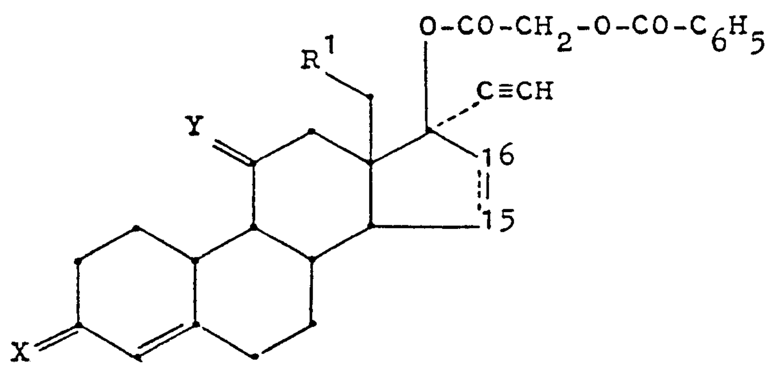

worin

X zwei Wasserstoffatome oder ein Sauerstoffatom,
Y zwei Wasserstoffatome oder eine Methylengruppe,
$R^1$ ein Wasserstoffatom oder eine Methylgruppe und
16 / 15 eine C-C-Einfach- oder Doppelbindung

bedeuten.

3. 17α-Ethinyl-17β-(O-benzoyl-glykoloyloxy)-4-östren-3-on (Norethisteronglykobenzoat).

**Patentansprüche** für den Vertragsstaat AT

1. Verfahren zur Herstellung einer wäßrigen Kristallsuspension eines 17-tertiären Steroidglykoesters enthaltend eine Verbindung der allgemeinen Formel I

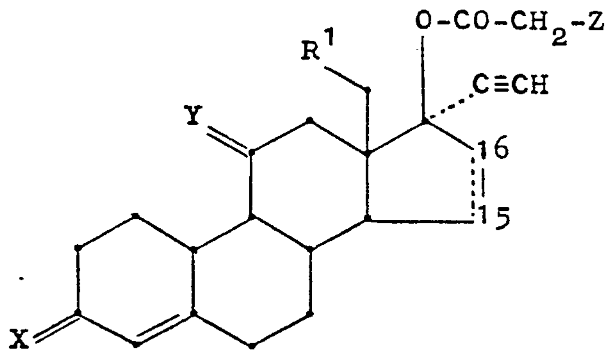

(I),

worin

X zwei Wasserstoffatome oder ein Sauerstoffatom,
Y zwei Wasserstoffatome oder eine Methylengruppe,
$R^1$ ein Wasserstoffatom oder eine Methylgruppe,
Z eine Hydroxygruppe oder die Gruppe -O-CO-$R^2$ mit $R^2$ in der Bedeutung einer Methyl- oder Phenylgruppe und
16 ⁞ 15 eine C-C-Einfach- oder Doppelbindung

bedeuten,
dadurch gekennzeichnet, daß man eine Suspension mit den folgenden Fraktionen

0 - 30 Gewichtsprozent der Teilchengröße 3 - 15 µm,
40 - 90 Gewichtsprozent der Teilchengröße 15 - 60 µm und
20 - 60 Gewichtsprozent der Teilchengröße 30 - 100 µm.

mit physiologischer Kochsalzlösung auffüllt.

2. Verfahren zur Herstellung von Glykobenzoesäureestern der allgemeinen Formel I a

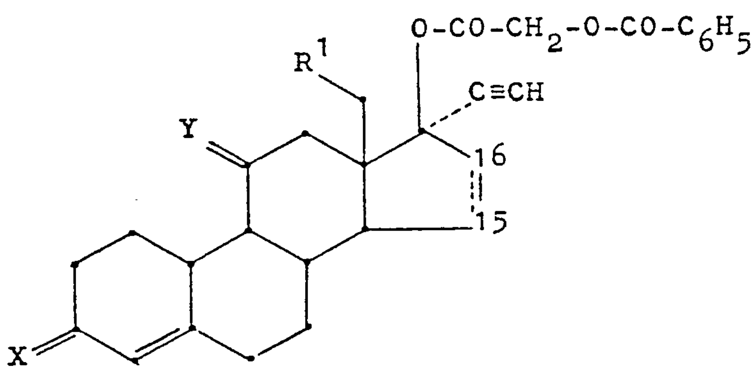

worin

X zwei Wasserstoffatome oder ein Sauerstoffatom,
Y zwei Wasserstoffatome oder eine Methylengruppe,
$R^1$ ein Wasserstoffatom oder eine Methylgruppe und
16 ⁞ 15 eine C-C-Einfach- oder Doppelbindung

bedeuten,
dadurch gekennzeichnet, daß man entsprechende Hydroxysteroide der allgemeinen Formel

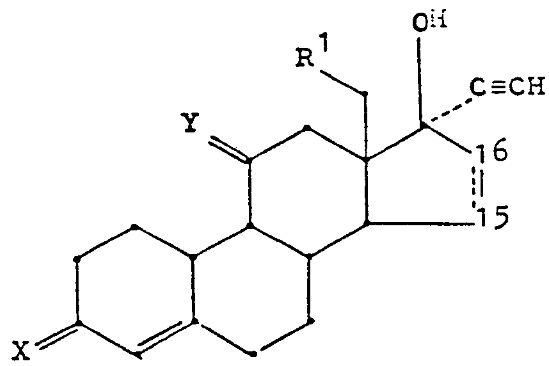

worin X, Y, $R^1$ und 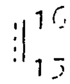 die oben angegebene Bedeutung haben, mit Benzoylglykoylchlorid verestert.

**Claims** for Contracting States BE, CH, LI, DE, FR, GB, IT, LU, NL, SE.

1. Aqueous crystalline suspension of a 17-tertiary steroid glycoester, characterized in that it contains a compound of general formula I

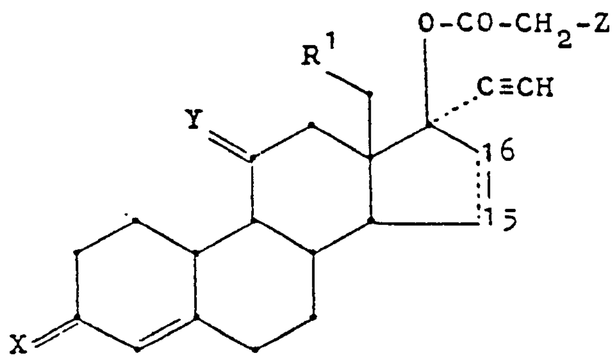

(I),

wherein

X means two hydrogen atoms or an oxygen atom,
Y means two hydrogen atoms or a methylene group,
$R^1$ means a hydrogen atom or a methyl group,
Z means a hydroxy group or the group -O-CO-$R^2$ wherein $R^2$ is a methyl or phenyl group, and
16
is a C-C- single or double bond,
15

in the following fractions:

0 - 30 % by weight of a particle size of 3 - 15 μm,
40 - 90 % by weight of a particle size of 15 - 60 μm, and
20 - 60 % by weight of a particle size of 30 - 100 μm.

2. Compounds of general formula Ia

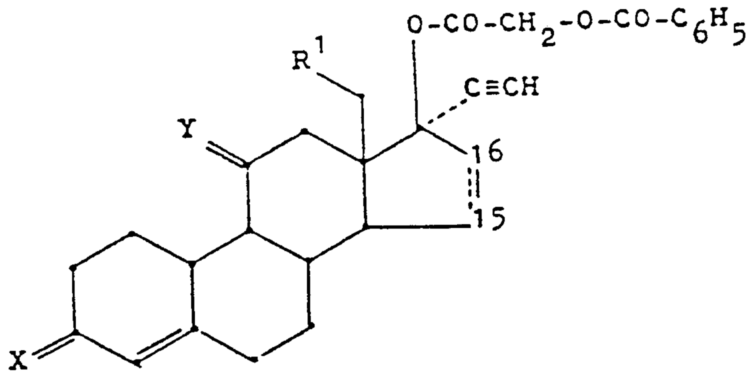

wherein

X means two hydrogen atoms or an oxygen atom,
Y means two hydrogen atoms or a methylene group,
$R^1$ means a hydrogen atom or a methyl group, and

16
means a C-C single or double bond.
15

3. 17α-ethynyl-17β-(O-benzoylglycoloyloxy)-4-estren-3-one (norethisterone glycobenzoate).

**Claims** for Contracting State AT

1. Process for the production of an aqueous crystalline suspension of a 17-tertiary steroid glycoester, containing a compound of general formula I

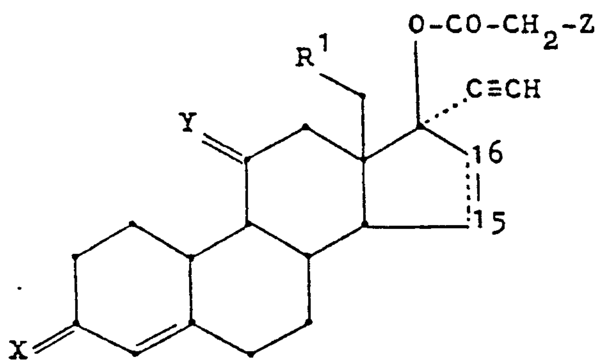

(I),

wherein

X means two hydrogen atoms or an oxygen atom,
Y means two hydrogen atoms or a methylene group,
$R^1$ means a hydrogen atom or a methyl group,
Z means a hydroxy group or the group $-O-CO-R^2$ wherein $R^2$ is a methyl or phenyl group, and

16
means a C-C single or double bond.
15

characterized in that a suspension containing the following fractions:

0- 30 % by weight of a particle size of 3 - 15 µm,
40 - 90 % by weight of a particle size of 15 - 60 µm, and
20 - 60 % by weight of a particle size of 30 - 100 µm,

is made up using physiological sodium chloride solution.

2. Process for the production of glycobenzoic acid esters of general formula Ia

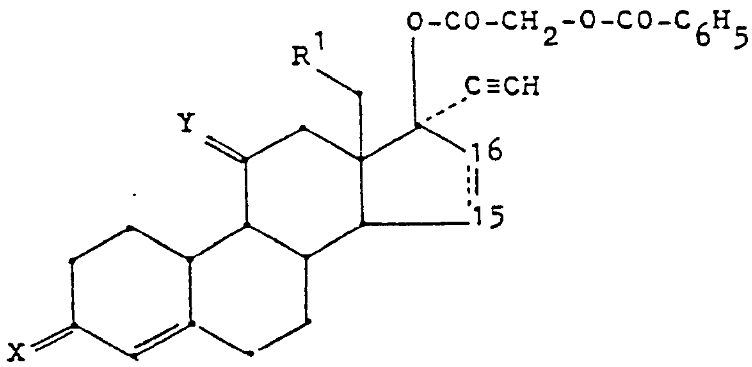

wherein

X means two hydrogen atoms or an oxygen atom,
Y means two hydrogen atoms or a methylene group,
$R^1$ means a hydrogen atom or a methyl group, and
$^{16}_{15}$ means a C-C single or double bond.

characterized in that corresponding hydroxy steroids of the general formula

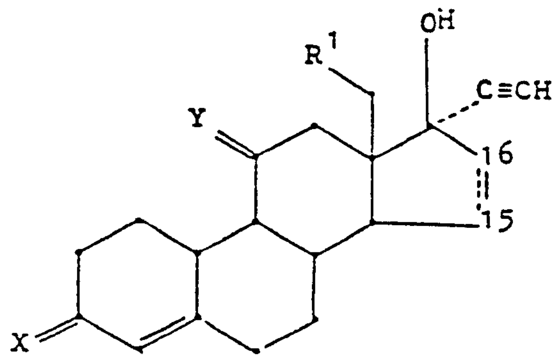

wherein X, Y, $R^1$ and $^{16}_{15}$ have the meanings given above, are esterified with benzoylglycoyl chloride.

**Revendications** pour les Etats contractants BE, CH, DE, FR, GB, IT, LI, LU, NL, SE

1. Suspension aqueuse de cristaux d'un ester glycolique de stéroïde sur le carbone 17 tertiaire, suspension caractérisée en ce qu'elle contient un composé répondant à la formule générale I:

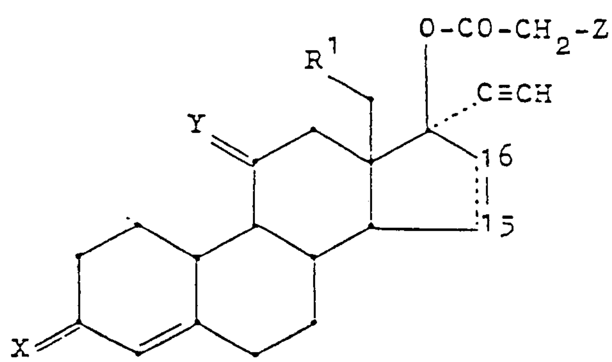

(I)

dans laquelle

X représente deux atomes d'hydrogène ou un atome d'oxygène,
Y représente deux atomes d'hydrogène ou un radical méthylène,
$R^1$ représente un atome d'hydrogène ou un radical méthyle,

Z représente un radical hydroxy ou un radical -O-CO-$R^2$ dans lequel $R^2$ désigne un radical méthyle ou phényle et

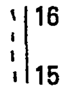 représente une liaison carbone-carbone simple ou double,

en les fractions suivantes:

de 0 à 30 % en poids de la dimension particulaire 3 - 15 µm,
de 40 à 90 % en poids de la dimension particulaire 15 - 60 µm et
de 20 à 60 % en poids de la dimension particulaire 30 - 100 µm.

2. Composés répondant à la formule générale Ia:

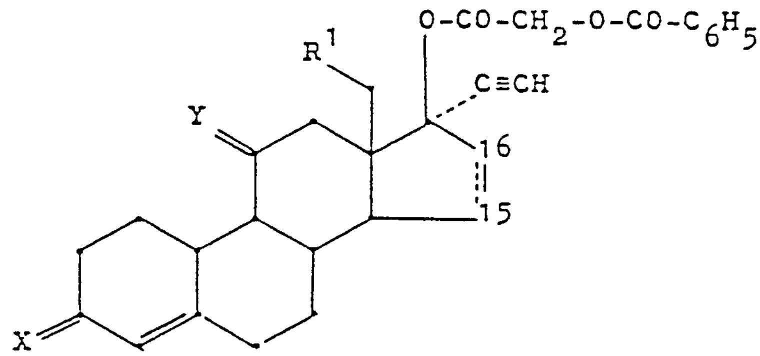

(Ia)

dans laquelle

X représente deux atomes d'hydrogène ou un atome d'oxygène,
Y représente deux atomes d'hydrogène ou un radical méthylène,
$R^1$ représente un atome d'hydrogène ou un radical méthyle et

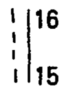 représente une liaison carbone-carbone simple ou double.

3. Ethynyl-17α-(O-benzoyl-glycoloyloxy)-17β estrène-4 one-3 (benzoyl-glycolate de noréthistérone).

**Revendications** pour l'Etat contractant AT

1. Procédé pour préparer une suspension aqueuse de cristaux d'un ester glycolique de stéroïde sur l'atome de carbone 17 tertiaire, qui contient un composé répondant à la formule I:

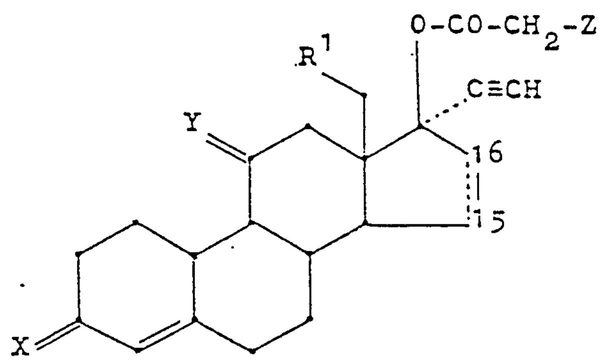

(I)

dans laquelle

X représente deux atomes d'hydrogène ou un atome d'oxygène,
Y représente deux atomes d'hydrogène ou un radical méthylène,
$R^1$ représente un atome d'hydrogène ou un radical méthyle,
Z représente un radical hydroxy ou un radical -O-CO-$R^2$ dans lequel $R^2$ désigne un radical méthyle ou phényle, et

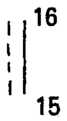 représente une liaison carbone-carbone simple ou double,

procédé caractérisé en ce qu'on étend avec une solution physiologique de chlorure de sodium une suspension comportant les fractions suivantes:

de 0 à 30 % en poids de la dimension particulaire 3 - 15 µm,
de 40 à 90 % en poids de la dimension particulaire 15 - 60 µm et
de 20 à 60 % en poids de la dimension particulaire 30 - 100 µm.

2. Procédé pour préparer des esters benzoyl-glycoliques répondant à la formule générale Ia:

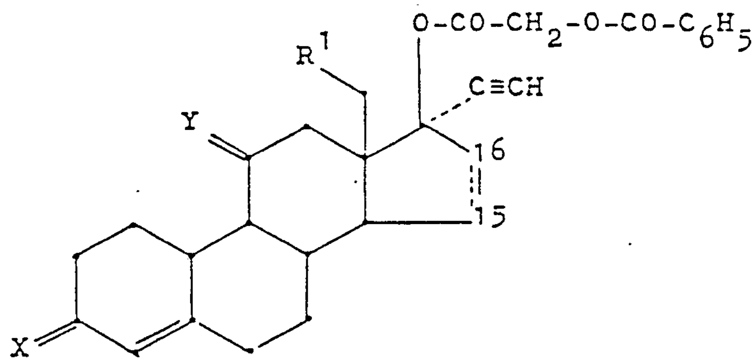

(Ia)

dans laquelle

X représente deux atomes d'hydrogène ou un atome d'oxygène,
Y représente deux atomes d'hydrogène ou un radical méthylène,
$R^1$ représente un atome d'hydrogène ou un radical méthyle et
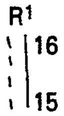 représente une liaison carbone-carbone simple ou double.

procédé caractérisé en ce qu'on estérifie des hydroxystéroïdes correspondants qui répondent à la formule générale:

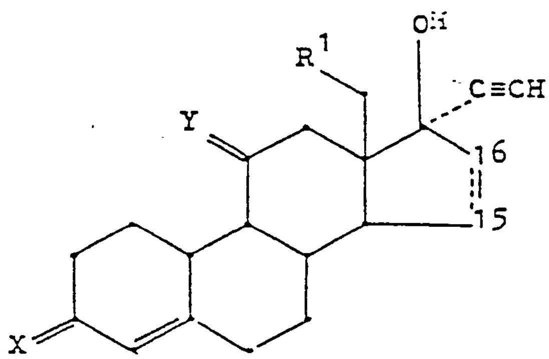

dans laquelle X, Y, $R^1$ et 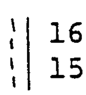 ont les significations indiquées plus haut, avec le chlorure de benzoyl-glycoloyle.

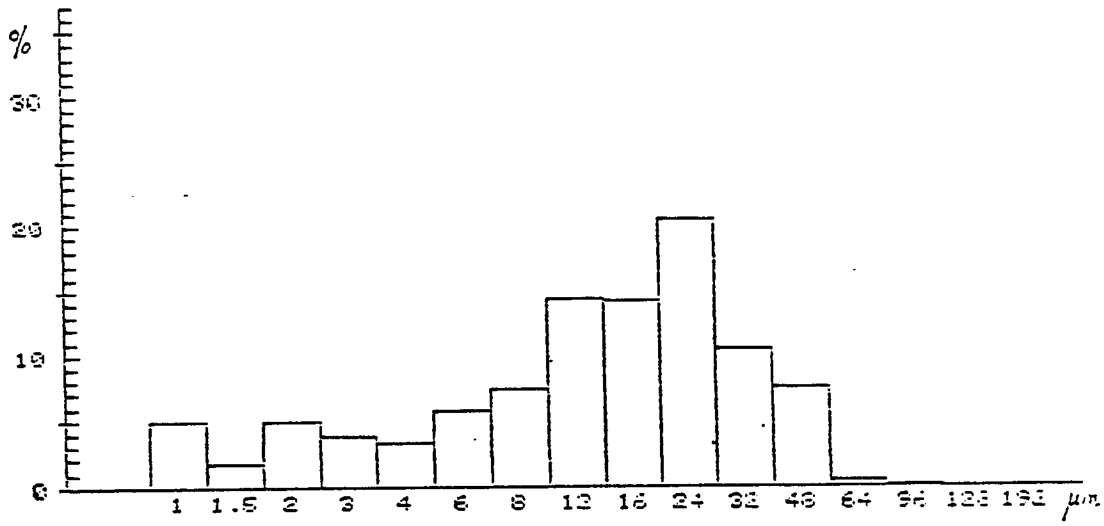


Abb. 1: Prozentuale Verteilung der Kristallgrößen in der geprüften Kristallsuspension von Norethisteronglycobenzoat

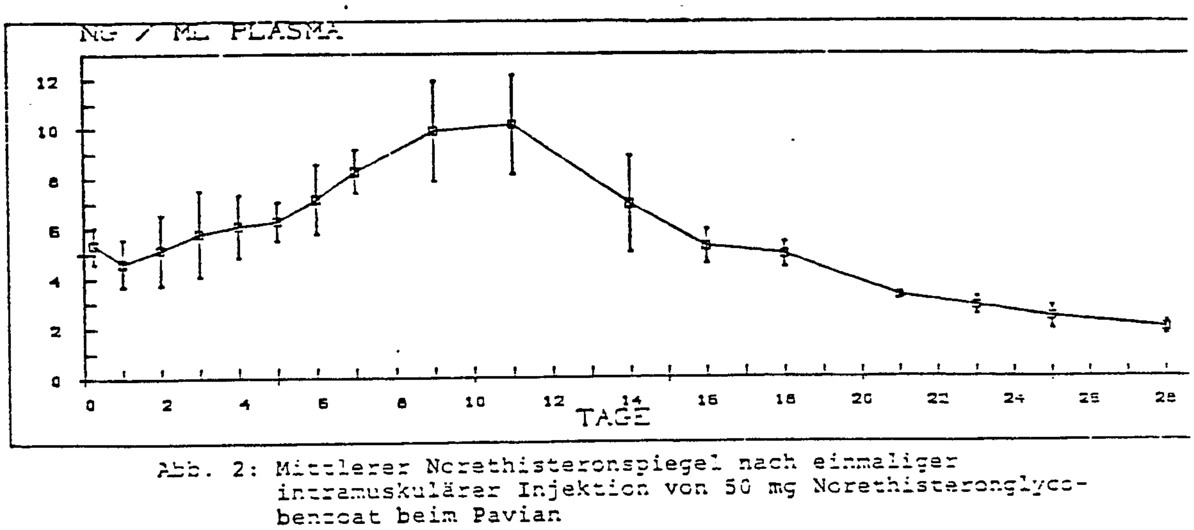


Abb. 2: Mittlerer Norethisteronspiegel nach einmaliger intramuskulärer Injektion von 50 mg Norethisteronglycobenzoat beim Pavian